# EUROPÄISCHE PATENTSCHRIFT

(11) **EP 1 021 428 B1**
(45) Veröffentlichungstag und Bekanntmachung des Hinweises auf die Patenterteilung: **25.07.2001**
(21) Anmeldenummer: 96938088.0
(22) Anmeldetag: 05.11.1996
(51) Int. Cl.: C07D 301/32

(54) **VERFAHREN ZUR DESTILLATION VON ETHYLENOXID**
PROCESS FOR DISTILLING ETHYLENE OXIDE
PROCEDE DE DISTILLATION D'OXYDE D'ETHYLENE

(30) Priorität: 17.11.1995 DE 19542829
(43) Veröffentlichungstag der Anmeldung: 26.07.2000
(73) Patentinhaber: BASF AKTIENGESELLSCHAFT, 67056 Ludwigshafen (DE)
(72) Erfinder: BESSLING, Bernd, D-67269 Grünstadt (DE); ZECK, Sebastian, D-67251 Freinsheim (DE); PLÜCKHAN, Jürgen, D-67227 Frankenthal (DE); MAYER, Thomas, D-67157 Wachenheim (DE); LÖFFLER, Ulrich, D-67125 Dannstadt-Schauernheim (DE); SPIEGEL, Günter, D-67549 Worms (DE); BALLENWEG, Rubens, D-69469 Weinheim (DE); BRUDI, Gerhard, D-67071 Ludwigshafen (DE); GIESELBERG, Klaus, D-67229 Laumersheim (DE); HILPRECHT, Lutz, D-67063 Ludwigshafen (DE); TERJUNG, Winfried, D-46149 Oberhausen (DE); AUER, Heinz, D-68809 Neulu heim (DE); POLT, Axel, Werner, D-67146 Deidesheim (DE); SCHOLL, Stephan, D-67098 Bad Dürkheim (DE)
(86) Internationale Anmeldenummer: EP9604818
(87) Internationale Veröffentlichungsnummer: WO9719069

(56) Entgegenhaltungen:
- DE-A- 2 850 254
- DE-A- 2 946 080
- CHEMIE-INGENIEUR-TECHNIK, Bd. 67, Nr. 12, Dezember 1995, Seiten 1614-1618, XP000542505 BESSLING B., LOEFFLER U., POLT A.: "Ethylenoxid-Reindestillation: Durch eine ganzheitliche Betrachtungsweise zu einem integrierten Verfahrens- und Sicherheitskonzept"
- ULLMANN'S ENCYCLOPEDIA OF INDUSTRIAL CHEMISTRY, Bd. A10, 1987, 5TH ED., Seiten 117-135, XP002024218 in der Anmeldung erwähnt

## Beschreibung

Die vorliegende Erfindung betrifft ein neues Verfahren zur Destillation von Ethylenoxid aus einem Ethylenoxid enthaltenden Gemisch in einer Kolonne, wobei man reines Ethylenoxid über Kopf oder über einen Seitenabzug im Verstärkungsteil der Kolonne abzieht.

Ethylenoxid kann durch Oxidation von Ethylen mit Sauerstoff hergestellt werden. Dabei findet die Oxidation in der Regel in einem Festbettreaktor bei einer Temperatur von 230 bis 270°C statt (Ullmann's Encyclopedia of Industrial Chemistry, 5 th Ed., Vol. A 10, Seiten 117 bis 135).

Der bei der Ethylenoxidherstellung resultierende Reaktionsaustrag der Ethylenoxid, Ethylen, Kohlendioxid, Acetaldehyd sowie Leichtsieder, wie Methan, enthält, wird mit Wasser behandelt, um auf diese Weise Ethylenoxid abzutrennen. Aus dem dabei erhaltenen Ethylenoxid und Wasser enthaltenden Gemisch kann nach Ausschleusung der Leichtsieder Ethylenoxid destillativ in reiner Form gewonnen werden.

Aufgrund seiner hohen Reaktivität ist Ethylenoxid ein bedeutendes Zwischenprodukt in der chemischen Industrie. Es dient beispielsweise zur Synthese von Ethylenglykol, Ethylenglykolethern, Oligo-oder Polyethylenglykolen oder Ethanolaminen.

Andererseits stellt die hohe Reaktivität des Ethylenoxids ein technisches Problem bei seiner Destillation dar, da es unter den dort herrschenden Bedingungen unter der Einwirkung einer Zündquelle explosionsartig zerfallen kann. Zudem kann es, wie bereits ausgeführt, mit dem ebenfalls vorhandenen Wasser zur Bildung von Glykolen kommen.

Aufgabe der vorliegenden Erfindung war es daher, ein neues Verfahren zur Destillation von Ethylenoxid aus einem Ethylenoxid enthaltenden Gemisch bereitzustellen, bei dem die Zerfallsreaktion von Ethylenoxid, insbesondere in ihrem Anfangsstadium, vollständig unterdrückt oder abgebrochen wird.

Es wurde nun gefunden, daß die Destillation von Ethylenoxid aus einem Ethylenoxid enthaltenden Gemisch in einer Kolonne bei einem absoluten Druck von 2 bis 10 bar und einer Temperatur von 20 bis 180°C, wobei man reines Ethylenoxid in flüssigem oder gasförmigem Aggregatzustand über Kopf oder über einen Seitenabzug im Verstärkungsteil der Kolonne abzieht, vorteilhaft gelingt, wenn man das Gemisch durch mehrere hintereinander angeordnete Zonen mit strukturierten Metallblechpackungen oder Schüttungen von Füllkörpern führt, von denen wenigstens eine mit einem Löschsystem verbunden ist, über das von außerhalb der Kolonne ein flüssiges, mit Ethylenoxid mischbares Löschmittel in die Zone(n) geleitet wird, sobald der Druck in der Kolonne einen Grenzwert übersteigt.

Vorzugsweise stammt das zu destillierende Gemisch aus der Ethylenoxidsynthese und enthält neben Ethylenoxid noch Wasser. Weitere Bestandteile können in diesem Fall z.B. Ethylenglykol, Oligoethylenglykole, Aldehyde, wie Formaldehyd oder Acetaldehyd, oder Methan sein.

Es ist aber auch möglich, solche Gemische im erfindungsgemäßen Verfahren zu destillieren, die neben Ethylenoxid z.B. Methanol, Glykole, Mono-, Di- oder Triethanolamin oder C₁-C₄-Mono- oder Dialkylethanolamine enthalten.

Bevorzugt enthält das zu destillierende Gemisch, jeweils bezogen auf sein Gewicht, 10 bis 90 Gew.-%, vorzugsweise 30 bis 70 Gew.-%, Ethylenoxid und 90 bis 10 Gew.-%, vorzugsweise 70 bis 30 Gew.-%, Wasser. Weiterhin kann das Gemisch, jeweils bezogen auf sein Gewicht, 0 bis 10 Gew.-%, vorzugsweise 0 bis 5 Gew.-% Ethylenglykol sowie 0 bis 10 Gew.-%, vorzugsweise 0 bis 5 Gew.-% Acetaldehyd enthalten. Es versteht sich, daß die Summe der Bestandteile des Gemisches jeweils 100 Gew.-% beträgt.

Erfindungsgemäß wird das neue Verfahren in einer Kolonne, vorzugsweise mit Verstärkungsteil, vorgenommen. Geeignete Kolonnen sind übliche, an sich bekannte Destillationskolonnen, wie sie z.B. in Ullmann's Encyclopedia of Industrial Chemistry, 5th Ed., Band B3, Seiten 4-82 bis 4-94, oder in Klaus Sattler "Thermische Trennverfahren: Grundlagen, Auslegung, Apparate", Verlag Chemie, 1988, Seiten 25 bis 27, beschrieben sind.

Die Kolonnen weisen in ihrem Inneren mehrere hintereinander angeordnete Zonen mit strukturierten Metallblechpackungen oder Schüttungen von Füllkörpern auf. Von zentraler Bedeutung ist, daß durch die besondere Führung des zu destillierenden Gemischs durch diese Zonen eine sehr gute destillative Trennwirkung erreicht wird und auch dem Zerfall von Ethylenoxid entgegen gewirkt werden kann. Es kann dabei von Vorteil sein, im Inneren der Kolonne zusätzliche Zonen mit strukturierten Metallblechpackungen oder Schüttungen von Füllkörpern vorzusehen, die nur als Zerfallssperren wirken.

Geeignete Füllkörper sind z.B. die an sich bekannten Raschigringe, Pallringe oder Bialeckiringe aus Metall oder Keramik als Trennmaterial.

Geeignete strukturierte Metallblechpackungen sind ebenfalls bekannt und beispielsweise unter der Bezeichnung Gempak® (Glitsch, Inc. Dallas, TX, U.S.A.), Mellapak® (Gebr. Sulzer, Winterthur, Schweiz) oder Relapak handelsüblich. Sie sind z.B. in Ullmann's Encyclopedia (loc. cit.) oder in Reinhard Billet "Packed Towers in Processing and Environmetal Technology", Verlag Chemie, Weinheim, 1995, Seiten 25 bis 27, beschrieben.

Neben diesen Zonen weist die Destillationskolonne noch an sich übliche Sammler- und Verteilersysteme auf.

Die spezifische Oberfläche einer Zone mit strukturierten Metallblechpackungen beträgt in der Regel 100 bis 1000 m², vorzugsweise 125 bis 750 m², jeweils bezogen auf einen m³ der Metallblechpackungszone.

Die spezifische Oberfläche einer Schüttungszone von Füllkörpern beträgt in der Regel 100 bis 1000 m², bezogen auf einen m³ der Schüttungszone.

Vorzugsweise nimmt man die Destillation in einer Kolonne vor, die mehrere Zonen von strukturierten Metallblechpackungen aufweist.

Besonders vorteilhaft führt man das neue Verfahren in einer Kolonne durch, die 2 bis 20, vorzugsweise 3 bis 15, insbesondere 4 bis 8 und ganz besonders bevorzugt 4 bis 6, dieser Zonen mit strukturierten Metallblechpackungen aufweist.

Die Höhe der einzelnen Packungszonen beträgt 1 bis 15 m, vorzugsweise 3 bis 8 m.

Die Zonen mit Metallblechpackungen oder Schüttungen von Füllkörpern sind mit einem oder mehreren außerhalb der Kolonne angebrachte Löschsystemen, die ein Löschmittel enthalten, verbunden.

Das Löschsystem, in der Regel ein mit dem Löschmittel gefüllter Behälter, der zusätzlich geringe Menge eines Inertgases, z. B. Stickstoff, enthalten kann, ist mittels Rohrleitungen und an sich bekannten Meß- und Regelvorrichtungen mit der Kolonne verbunden. Das Löschmittel kann dabei z.B. nach dem Öffnen von Ventilen mittels Pumpen in die Kolonne geleitet werden. In einer bevorzugten Ausführungsform des erfindungsgemäßen Verfahrens sind die Löschsysteme oberhalb der obengenannten Zonen angebracht. Man macht sich auf diese Weise den hydrostatischen Druck des Löschmittels beim Löschvorgang zunutze.

Zur optimalen Verteilung des Löschmittels in der Kolonne können vorteilhaft die in der Kolonne befindlichen üblichen Sammler- und Verteilersysteme verwendet werden.

Als Löschmittel können Flüssigkeiten, die mit Ethylenoxid mischbar sind, verwendet werden. Vorzugsweise verwendet man ein wäßriges Medium als Löschmittel. Insbesondere ist Wasser als Löschmittel zu nennen. Das Löschwasser (z.B. Kühlwasser oder Flußwasser) kann dabei noch Hilfsmittel, z.B. Frostschutzmittel, enthalten.

Ein explosionsartiger Zerfall von gasförmigem Ethylenoxid ist mit einem Druckanstieg bis zum Zehnfachen des Anfangsdruckes verbunden. Findet ein Zerfall von flüssigem Ethylenoxid statt, kann durch die entstehenden Zerfallsgase ein noch höherer Druck erreicht werden. Beim Überschreiten eines einstellbaren Grenzwertes für den Druck wird der Löschvorgang ausgelöst. Im allgemeinen liegt der Grenzwert dabei 0,01 bis 4 bar, vorzugsweise 0,1 bis 2 bar, besonders bevorzugt 0,2 bis 1 bar und insbesondere ca. 0,5 bar über dem Betriebsdruck in der Kolonne. Nach einem Zeitraum von ca. 5 bis 60 Sekunden, vorzugsweise ca. 15 Sekunden tritt das Löschmittel in die Packungszonen ein.

Bei einem Zerfall der nach unten fortschreitet, bewegt sich die Zerfallsfront schneller als die Löschfront. Der Zerfall wird hier erst in der darunterliegenden Packungszone gestoppt, da dieser Bereich bereits mit Löschmittel gespült wurde und deshalb die dort herrschenden Bedingungen keinen weiteren Zerfall von Ethylenoxid mehr zulassen. Bei einem Zerfall, der sich nach oben bewegt, laufen die Zerfallsfront und die Löschfront aufeinander zu und treffen aufeinander, woraus dann der Löschvorgang resultiert.

Bevorzugt wird beim Löschvorgang soviel Löschmittel, vorzugsweise Wasser, in die Kolonne eingeleitet, daß das Gewichtsverhältnis Ethylenoxid:Löschmittel in dem zu löschenden Bereich zwischen zwei Packungszonen bei der Löschung unterhalb von 80:20, vorzugsweise unterhalb von 70:30, liegt.

Bevorzugt ist eine Verfahrensweise, bei der jede Zone mit Metallblechpackungen oder Schüttungen von Füllkörpern mit einem separaten Löschsystem verbunden ist.

Es ist von Vorteil, den Löschmittelbehälter des obersten Löschsystems größer auszulegen als die Behälter der restlichen Löschsysteme. Bei ca. 1,5- bis 2-facher Auslegung dieses Behälters ist sichergestellt, daß ein Nachlauf von Löschmittel in der gesamten Kolonne erfolgen kann, wodurch der Löschvorgang zusätzlich intensiviert wird.

Im erfindungsgemäßen Verfahren, das in kontinuierlicher Arbeitsweise durchgeführt wird, wird das zur Destillation gelangende, Ethylenoxid enthaltende Gemisch in ca. 30 bis 80 %, vorzugsweise ca. 50 %, der Kolonnenhöhe, jeweils gemessen vom Fuß der Kolonne, in die Kolonne geleitet. Das Rücklaufverhältnis beträgt 1,5:1 bis 6:1, vorzugsweise 2,5:1 bis 3,5:1. Die Destillation wird bei einem absoluten Druck von 2 bis 10 bar, vorzugsweise 2,5 bis 4 bar, und bei einer Temperatur von 10 bis 180°C, vorzugsweise 30 bis 150°C, vorgenommen.

Reines Ethylenoxid wird als Kopfabzug oder über einen Seitenabzug im Verstärkungsteil der Kolonne abgezogen.

Bevorzugt ist eine Verfahrensweise, bei der reines Ethylenoxid in gasförmigem Aggregatzustand über Kopf oder in gasförmigem oder flüssigem Aggregatzustand über einen Seitenabzug abgezogen wird.

Wenn Ethylenoxid über einen Seitenabzug im Verstärkungsteil der Kolonne abgezogen wird, so können vorteilhaft Leichtsieder, z.B. Formaldehyd, als Kopfprodukt abgezogen werden.

Bei der Destillation eines Ethylenoxid und Wasser enthaltenden Gemisches wird in der Regel Wasser im Sumpf der Kolonne abgezogen.

Bei der Destillation eines Ethylenoxid, Wasser und Acetaldehyd enthaltenden Gemisches wird vorzugsweise reines Ethylenoxid über Kopf der Kolonne, ein Gemisch, enthaltend Acetaldehyd, Ethylenoxid und Wasser, über einen Seitenabzug im Abtriebsteil der Kolonne und Wasser im Sumpf der Kolonne abgezogen.

Bei der Destillation eines Ethylenoxid, Wasser, Formaldehyd und Acetaldehyd enthaltenden Gemisches wird vorzugsweise ein Gemisch aus Formaldehyd und Ethylenoxid über Kopf der Kolonne, reines Ethylenoxid über einen Seitenabzug im Verstärkungsteil der Kolonne, ein Gemisch, enthaltend Acetaldehyd, Ethylenoxid und Wasser, über einen Seitenabzug im Abtriebsteil der Kolonne und Wasser im Sumpf der Kolonne abgezogen.

Die zur Destillation benötigte Wärmemenge kann auf verschiedene Weise zugeführt werden. Beispielsweise kann sie bei der Destillation eines Ethylenoxid und Wasser enthaltenden Gemisches im Sumpf der Kolonne, im Abtriebsteil der Kolonne oder durch Erwärmen des zu destillierenden Gemisches (Feed) zugeführt werden. Bei der Destillation von einem Ethylenoxid und Wasser enthaltenden Gemisch ist es von besonderem Vorteil, die Wärmezufuhr im Abtriebsteil der Kolonne oder durch Erwärmen der Feeds vorzunehmen, da durch das wäßrige System inhärent ein Löschmittel vorhanden ist, das dem Zerfall von Ethylenoxid entgegenwirkt.

Eine besonders bevorzugte Verfahrensweise besteht darin, die Destillation in einer Kolonne vorzunehmen, die ummantelt ist, wobei der Zwischenraum zwischen Kolonne und Mantel mit einem inerten Gas, z.B. Kohlendioxid oder Stickstoff, vorzugsweise Stickstoff, gespült wird. Mit diesen Maßnahmen läßt sich ein zusätzlicher Schutz gegenüber externen Zündquellen, die den explosionsartigen Zerfall von Ethylenoxid initiieren können, erreichen.

Mittels des neuen Verfahrens gelingt es in vorteilhafter Weise, Ethylenoxid in kontinuierlicher Arbeitsweise aus einem Ethylenoxid enthaltenden Gemisch zu destillieren, wobei sichergestellt ist, daß wenn ein spontaner Zerfall von Ethylenoxid erfolgt, dieser sofort unter Kontrolle gebracht werden kann, ohne daß es zur explosionsbedingten Zerstörung der Destillationsanlage kommt. Die Reinheit des im erfindungsgemäßen Verfahren erhaltenen Ethylenoxid ist sehr hoch. Beispielsweise erhält man bei der Destillation eines bei der Synthese von Ethylenoxid anfallenden wäßrigen, Ethylenoxid enthaltenden Reaktionsgemisches Ethylenoxid, dessen Gehalt an Acetaldehyd höchstens 30 ppm, der an Wasser höchstens 60 ppm beträgt.

## Patentansprüche

1. Verfahren zur Destillation von Ethylenoxid aus einem Ethylenoxid enthaltenden Gemisch in einer Kolonne bei einem absoluten Druck von 2 bis 10 bar und einer Temperatur von 20 bis 180°C, wobei man reines Ethylenoxid in flüssigem oder gasförmigem Aggregatzustand über Kopf oder über einen Seitenabzug im Verstärkungsteil der Kolonne abzieht, dadurch gekennzeichnet, daß man das Gemisch durch mehrere hintereinander angeordnete Zonen mit strukturierten Metallblechpackungen oder Schüttungen von Füllkörpern führt, die sich im Inneren der Kolonne befinden und von denen wenigstens eine mit einem Löschsystem verbunden ist, über das von außerhalb der Kolonne ein flüssiges, mit Ethylenoxid mischbares Löschmittel in die Zone(n) geleitet wird, sobald der Druck in der Kolonne einen Grenzwert übersteigt.

2. Verfahren nach Anspruch 1, dadurch gekennzeichnet, daß man ein Gemisch destilliert, das Ethylenoxid und Wasser enthält.

3. Verfahren nach Anspruch 1, dadurch gekennzeichnet, daß man ein Gemisch destilliert, das, bezogen auf sein Gewicht, 10 bis 90 Gew.-% Ethylenoxid und 90 bis 10 Gew.-% Wasser enthält.

4. Verfahren nach Anspruch 1, dadurch gekennzeichnet, daß die spezifische Oberfläche einer Zone mit strukturierten Metallblechpackungen 100 bis 1000 m² je m³ der Metallpackungszone beträgt.

5. Verfahren nach Anspruch 1, dadurch gekennzeichnet, daß die spezifische Oberfläche einer Zone mit Schüttungen von Füllkörpern 100 bis 1000 m² je m³ der Schüttungszone beträgt.

6. Verfahren nach Anspruch 1, dadurch gekennzeichnet, daß man die Destillation in einer Kolonne vornimmt, die mehrere Zonen mit strukturierten Metallblechpackungen aufweist.

7. Verfahren nach Anspruch 1, dadurch gekennzeichnet, daß man die Destillation in einer Kolonne durchführt, die 2 bis 20 Zonen mit strukturierten Metallblechpackungen aufweist.

8. Verfahren nach Anspruch 1, dadurch gekennzeichnet, daß jede Zone mit Metallblechpackungen oder Schüttungen von Füllkörpern mit einem separaten Löschsystem verbunden ist.

9. Verfahren nach Anspruch 1, dadurch gekennzeichnet, daß man ein wäßriges Medium als Löschmittel verwendet.

10. Verfahren nach Anspruch 1, dadurch gekennzeichnet, daß das Löschmittel in die Zone(n) geleitet wird, sobald der Druck in der Kolonne den Betriebsdruck um 0,01 bis 4 bar übersteigt.

## Claims

1. A process for distilling ethylene oxide out of a mixture comprising ethylene oxide in a column at an absolute pressure of from 2 to 10 bar and a temperature of 20 to 180°C by withdrawing pure ethylene oxide in the liquid or gaseous state overhead or via a sidestream in the rectifying section of the column, which comprises feeding the mixture through a plurality of successive zones of structured sheet-metal packing or dumped beds of packing elements, which zones are present on the inside of the column and at least one of which zones is connected to an extinguishing system via which a liquid solvent which is miscible with ethylene oxide is fed into the zone(s) from outside the column as soon as the pressure limit in the column is exceeded.

2. A process as claimed in claim 1, wherein the mixture to be distilled comprises ethylene oxide and water.

3. A process as claimed in claim 1, wherein the mixture to be distilled comprises, based on its weight, from 10 to 90% by weight of ethylene oxide and from 90 to 10% of water.

4. A process as claimed in claim 1, wherein the specific surface area of a zone of structured sheet-metal packing is from 100 to 1000 m² per m³ of the metal-packing zone.

5. A process as claimed in claim 1, wherein the specific surface area of a zone of dumped beds of packing elements is from 100 to 1000 m² per m³ of the dumped-packing zone.

6. A process as claimed in claim 1, wherein the distillation is effected in a column having a plurality of zones of structured sheet-metal packing.

7. A process as claimed in claim 1, wherein the distillation is carried out in a column having from 2 to 20 zones of structured sheet-metal packing.

8. A process as claimed in claim 1, wherein each metal-packing or dumped-packing zone is connected to a separate extinguishing system.

9. A process as claimed in claim 1, wherein the extinguishant used is an aqueous medium.

10. A process as claimed in claim 1, wherein the solvent is passed into the zone(s) as soon as the pressure in the column exceeds the operating pressure by from 0.01 to 4 bar.

## Revendications

1. Procédé pour la distillation de l'oxyde d'éthylène à partir d'un mélange contenant de l'oxyde d'éthylène, dans une colonne à une pression absolue de 2 à 10 bars et à une température de 20 à 180°C, dans lequel on retire de l'oxyde d'éthylène pur à l'état d'agrégat liquide ou gazeux par la tête ou par un soutirage latéral dans la partie à concentration de la colonne, caractérisé en ce que, l'on conduit le mélange à travers plusieurs zones disposées l'une derrière l'autre, avec des garnissages de tôle métallique structurés ou des remplissages de corps de garnissage, qui se trouvent à l'intérieur de la colonne, et dont au moins une est reliée à un système d'extinction, par lequel on introduit depuis l'extérieur de la colonne, une matière extinctrice liquide miscible à l'oxyde d'éthylène dans la ou les zones, dès que la pression dans la colonne dépasse une valeur limite.

2. Procédé selon la revendication 1, caractérisé en ce que l'on distille un mélange qui contient de l'oxyde d'éthylène et de l'eau.

3. Procédé selon la revendication 1, caractérisé en ce que l'on distille un mélange qui contient 10 à 90% en poids d'oxyde d'éthylène et 90 à 10% en poids d'eau par rapport à son poids.

4. Procédé selon la revendication 1, caractérisé en ce que la surface spécifique d'une zone ayant des garnissages de tôle métallique structurés est de 100 à 1 000 m²/m³ de la zone de garnissage métallique.

5. Procédé selon la revendication 1, caractérisé en ce que la surface spécifique d'une zone avec des remplissages de corps de garnissage est de 100 à 1 000 m²/m³ de zone de remplissage.

6. Procédé selon la revendication 1, caractérisé en ce que l'on procède à la distillation dans une colonne qui comporte plusieurs zones avec des garnissages de tôle métallique structurés.

7. Procédé selon la revendication 1, caractérisé en ce que l'on réalise la distillation dans une colonne qui comporte 2 à 20 zones ayant des garnissages de tôle métallique structurés.

8. Procédé selon la revendication 1, caractérisé en ce que chaque zone ayant des garnissages de tôle métallique ou des remplissages de corps de garnissage est reliée à un système d'extinction séparé.

9. Procédé selon la revendication 1, caractérisé en ce que l'on utilise un milieu aqueux comme matière extinctrice.

10. Procédé selon la revendication 1, caractérisé en ce que l'on introduit la matière extinctrice dans la ou les zones, dès que la pression dans la colonne dépasse la pression de fonctionnement de 0,01 à 4 bars.
